(19) **Europäisches Patentamt**

**European Patent Office**

**Office européen des brevets**

(11) **EP 0 721 772 B1**

(12) **EUROPÄISCHE PATENTSCHRIFT**

(45) Veröffentlichungstag und Bekanntmachung des Hinweises auf die Patenterteilung:
**08.11.2000  Patentblatt 2000/45**

(51) Int. Cl.[7]: **A61K 7/09**

(21) Anmeldenummer: **95113584.7**

(22) Anmeldetag: **30.08.1995**

(54) **Verwendung eines Mittels und Verfahren zur dauerhaften Haarverformung**

Use of an agent and process for permanent hair waving

Utilisation d'un agent et procédé pour la déformation permanente des cheveux

(84) Benannte Vertragsstaaten:
**DE ES FR GB IT**

(30) Priorität: **12.01.1995 DE 19500680**

(43) Veröffentlichungstag der Anmeldung:
**17.07.1996  Patentblatt 1996/29**

(73) Patentinhaber:
**Wella Aktiengesellschaft**
**64274 Darmstadt (DE)**

(72) Erfinder:
• **Lang, Günther, Dr.**
  **D-64354 Reinheim (DE)**
• **Bruhn, Frank-Rainer Dr.**
  **D-64295 Darmstadt (DE)**

(56) Entgegenhaltungen:
**EP-A- 0 362 663**       **EP-A- 0 395 332**
**WO-A-94/14428**

**Beschreibung**

Die vorliegende Erfindung betrifft die Verwendung eines Mittels zur dauerhaften Haarverformung, welches als keratinreduzierenden Wirkstoff 2,3-Dimercaptobernsteinsäure enthält, sowie ein Verfahren zur dauerhaften Haarverformung unter Verwendung dieses Mittels.

Bekanntlich besteht die klassische Technik zur Durchführung der dauerhaften Haarverformung darin, daß in einer ersten Stufe die Disulfidbindungen des Haarkeratins mit Hilfe eines Mittels, welches einen reduzierenden Wirkstoff enthält (Haarverformungsmittel), geöffnet werden, sodann das Haar in die gewünschte Form gebracht wird und anschließend die Disulfidbindung unter Verwendung eines einen oxidierenden Wirkstoff enthaltenden Mittels (Fixiermittel) wieder verknüpft werden,

Als reduzierende Wirkstoffe werden hierbei insbesondere Sulfite, Thioglykolsäure Thiomilchsäure, 3-Mercaptopropionsäure, Mercaptocarbonsäureester, Cystein und dessen Derivate oder Cysteamin und dessen Derivate verwendet.

Aus der WO-A-94 14428 S.5 Tabelle 1 ist eine zur Verhinderung von Haarwachstum bestimmte Zubereitung (Hautbehandlungsmittel) mit einem Gehalt an 20 % meso-2,3-Dimercaptobernsteinsäure bekannt.

Es ist bekannt, daß Mercaptocarbonsäureester, wie zum Beispiel Thioglykolsäureglycerinester, bezüglich ihrer Hautverträglichkeit sowie ihres Sensibilisierungsrisikos nicht zufriedenstellend sind, während die Thioglykolsäure eine vergleichsweise hohe Toxizität aufweist. Ein weiteres Problem stellt der typische intensive Thiolgeruch der genannten Reduktionsmittel dar, der eine starke Parfümierung der Produkte erfordert.

Es bestand daher die Aufgabe, ein neues Verfahren auf der Basis von keratinreduzierenden Verbindungen zur Verfügung zu stellen, welche eine gute Hautverträglichkeit, ein geringes Sensibilisierungsrisiko sowie eine niedrige Toxizität und eine gute biologische Abbaubarkeit bei gleichzeitig guter Verformungswirksamkeit aufweisen. Darüber hinaus bestand ein Bedarf an Reduktionsmitteln, durch die die bekannten Geruchsprobleme bei der Dauerwellbehandlung beziehungsweise in dauergewelltem Haar vermieden werden können.

Überraschenderweise wurde nunmehr gefunden, daß durch die Verwendung von 2,3-Dimercaptobernsteinsäure beziehungsweise deren Salze enthaltender Mittel eine wirkungsvolle Umformung der Haare bei gleichzeitig guter physiologischer Verträglichkeit und geringem Sensibilisierungsrisiko ermöglicht wird, ohne daß der sehr unangenehme Mercapto-Geruch auftritt.

Gegenstand der vorliegenden Erfindung ist daher die Verwendung eines Mittels auf der Basis eines keratinreduzierenden Wirkstoffes, welches als haarkeratinreduzierenden Wirkstoff 2,3-Dimercaptobernsteinsäure und/oder deren Salze enthält, wobei sowohl die meso- als auch die D-, L- und DL-Form geeignet sind zur dauerhaften Verformung von Haaren.

Als Salze der 2,3-Dimercaptobernsteinsäure eignen sich insbesondere die Alkalisalze, das Ammoniumsalz und das Monoethanolaminsalz der 2,3-Dimercaptobernsteinsäure, wobei das Ammoniumsalz und das Monoethanolaminsalz der 2,3-Dimercaptobernsteinsäure bevorzugt sind.

Zwar ist es möglich, 2,3-Dimercaptobernsteinsäure und/oder deren Salze gemeinsam mit anderen keratinreduzierenden Wirkstoffen - wie zum Beispiel Thioglykolsäure, Thiomilchsäure, 3-Mercaptopropionsäure, Cysteamin oder Cysteaminderivaten und Cystein oder Cysteinderivaten - zu verwenden, jedoch ist die Verwendung von 2,3-Dimercaptobernsteinsäure und/oder deren Salze als alleinigem keratinreduzierenden Wirkstoff (das heißt ohne Zusatz anderer keratinreduzierender Wirkstoffe) besonders bevorzugt.

Die 2,3-Dimercaptobernsteinsäure und/oder deren Salze werden in dem gebrauchsfertigen Mittel zur dauerhaften Haarverformung in einer Menge von 3 bis 25 Gewichtsprozent, vorzugsweise in einer Menge von 8 bis 20 Gewichtsprozent, eingesetzt.

Das gebrauchsfertige Verformungsmittel besitzt einen pH-Wert von 6 bis 9, vorzugsweise von 6,5 bis 8,7.

Das Verformungsmittel kann sowohl in Form einer wäßrigen Lösung oder einer Emulsion als auch in verdickter Form auf wäßriger Basis, insbesondere als Gel, Creme oder Paste vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; oder Alkohole, wie beispielsweise Ethanol, Propanol, Isopropanol oder Glycerin; Lösungsvermittler; Stabilisatoren; Puffersubstanzen; Parfümöle, Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,2 bis 30 Gewichtsprozent, während die Verdickungsmittel in einer Menge von 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 2 bis 30 Gewichtsprozent sowie zur Vermeidung einer Überkrausung der Haare Dithioverbindungen, beispielsweise Dithiodiglykolsäure, Dithiodimilchsäure oder deren Salze, zugesetzt werden.

Durch Variation des pH-Wertes kann ein Mittel für die erfindungsgemäße Verwendung zur Verfügung gestellt werden, das universell für jede Haarstruktur, gegebenenfalls unter zusätzlicher Wärmeeinwirkung, geeignet ist. Das Mittel bewirkt eine elastische, dauerhafte, gleichmäßige Umformung vom Haaransatz bis zur Haarspitze, ohne allergische oder sensibilisierende Reaktionen hervorzurufen.

Die vorliegende Erfindung betrifft weiterhin ein Verfahren zur dauerhaften Verformung von Haaren, bei dem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet, welches dadurch gekennzeichnet ist, daß als Verformungsmittel das vorstehend beschriebene Mittel verwendet wird.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Beispiele

Beispiel 1: Dauerverformungsmittel für gefärbtes Haar

| | |
|---|---|
| 10,0 g | 2,3-Dimercaptobernsteinsäure |
| 6,1 g | Ammoniak (25%-ige wäßrige Lösung) |
| 2,0 g | Ammoniumhydrogencarbonat |
| 2,0 g | Isopropanol |
| 1,0 g | Isooctylphenol, oxethyliert mit 10 Mol Ethylenoxid |
| 1,0 g | Poly(dimethyldiallylammoniumchlorid) |
| 0,3 g | Parfümöl |
| 0,1 g | Vinylpyrrolidon/Styrol-Mischpolymerisat (Antara**®** 430 der GAF Corp.; New York/USA) |
| 77,5 g | Wasser |
| 100,0 g | |

Der pH-Wert dieses Mittels beträgt 8,0.

Durch Farbbehandlungen vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 40 Grad Celsius erwärmt.

Anschließend wird die Abdeckung entfernt, das Haar mit Wasser gespült und mit 100 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und sodann getrocknet.

Als Ergebnis dieser Behandlung wird eine gleichmäßige, elastische und dauerhafte Verformung der Haare erhalten.

Beispiel 2: Dauerverformungsmittel für normales Haar

| 16,0 g | 2,3-Dimercaptobernsteinsäure |
|--------|------------------------------|
| 8,9 g | Ammoniak (25%-ige wäßrige Lösung) |
| 5,0 g | Ammoniumhydrogencarbonat |
| 4,0 g | Harnstoff |
| 2,4 g | Monoethanolamin |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat (Antara® 430 der GAF Corp.; New York/USA) |
| 61,1 g | Wasser |
| 100,0 g | |

Der pH-Wert dieses Mittels beträgt 8,4.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet.

Das so behandelte Haar besitzt eine gleichmäßige und lebhafte Krause.

Beispiel 3: Dauerverformungsmittel für normales und schwer verformbares Haar

| 3,0 g | 2,3-Dimercaptobernsteinsäure |
|-------|------------------------------|
| 15,0 g | Cysteinhydrochlorid |
| 11,0 g | Ammoniak (25%-ige wäßrige Lösung) |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat (Antara® 430 der GAF Corp.; New York/USA) |
| 68,4 g | Wasser |
| 100,0 g | |

Der pH-Wert dieses Mittels beträgt 8,7.

Das Haar wird in der in Beispiel 2 beschriebenen Weise behandelt, wobei die Einwirkungszeit des Verformungsmittels jedoch 20 Minuten beträgt.

Das Ergebnis dieser Behandlung ist eine natürlich wirkende, gleichmäßige Umformung der Haare vom Haaransatz bis zu den Haarspitzen.

Alle in der vorliegenden Anmeldung enthaltenen Prozentangaben stellen, soweit nicht anders angegeben, Gewichtsprozent dar.

**Patentansprüche**

1. Verfahren zur dauerhaften Verformung von Haaren, bei dem man das Haar bevor oder nachdem man es in die gewünschte Form bringt mit einem Verformungsmittel behandelt, mit Wasser spült, sodann oxidativ nachbehandelt, mit Wasser spült, gegebenenfalls zur Wasserwelle legt und sodann trocknet, dadurch gekennzeichnet, daß man als Verformungsmittel ein Mittel zur dauerhaften Verformung von Haaren auf der Basis eines keratinreduzierenden Wirkstoffes verwendet, das als keratinreduzierenden Wirkstoff 2,3-Dimercaptobernsteinsäure und/oder deren Salze enthält.

2. Verfahren nach Anspruch 1, dadurch gekennzeichnet, daß das Salz der 2,3-Dimercaptobernsteinsäure ausgewählt ist aus dem Ammoniumsalz und dem Monoethanolaminsalz der 2,3-Dimercaptobernsteinsäure.

3. Verfahren nach Anspruch 1 oder 2, dadurch gekennzeichnet, daß die 2,3-Dimercaptobernsteinsäure und/oder deren Salze in dem gebrauchsfertigen Mittel in einer Menge von 3 bis 25 Gewichtsprozent enthalten sind.

4. Verfahren nach einem der Ansprüche 1 bis 3, dadurch gekennzeichnet, daß der pH-Wert des gebrauchsfertigen Mittels 6 bis 9 beträgt.

5. Verfahren nach einem der Ansprüche 1 bis 4 dadurch gekennzeichnet, daß das Verformungsmittel als alleinigen keratinreduzierenden Wirkstoff 2,3-Dimercaptobernsteinsäure und/oder deren Salze enthält.

6. Verfahren nach einem der Ansprüche 1 bis 4, dadurch gekennzeichnet, daß es neben der 2,3-Dimercaptobernsteinsäure und/oder deren Salzen weitere keratinreduzierende Verbindungen enthält.

7. Verfahren nach Anspruch 6, dadurch gekennzeichnet, daß die zusätzliche keratinreduzierende Verbindung ausgewählt ist aus Thioglykolsäure, Thiomilchsäure, 3-Mercaptopropionsäure, Cystein oder dessen Derivaten und Cysteamin oder dessen Derivaten.

8. Verfahren nach Anspruch 7, dadurch gekennzeichnet, daß man das Verformungsmittel 5 bis 30 Minuten lang einwirken läßt.

**9.** Verfahren nach Anspruch 7 oder 8, dadurch gekennzeichnet, daß man das Verformungsmittel in einer Menge von 60 bis 120 Gramm anwendet.

**10.** Verwendung eines Mittels auf der Basis eines keratinreduzierenden Wirkstoffes, das als keratinreduzierenden Wirkstoff 2,3-Dimercaptobernsteinsäure und/oder deren Salze enthält, zur dauerhaften Verformung von Haaren.

**Claims**

**1.** Process for the permanent shaping of hair in which the hair, before or after its is brought into the desired shape, is treated with a shaping agent, rinsed with water, then aftertreated oxidatively, rinsed with water, optionally followed by a water wave, then dried, characterised in that the shaping agent is an agent for permanent shaping hair, based on a keratin-reducing active substance which contains 2,3-dimercaptosuccinic acid and/or its salts as the keratin-reducing active substance.

**2.** Process according to Claim 1, characterised in that the salt of 2,3-dimercaptosuccinic acid is selected from the ammonium salt and the monoethanolamine salt of 2,3-dimercaptosuccinic acid.

**3.** Process according to Claim 1 or 2, characterised in that the 2,3-dimercaptosuccinic acid and/or its salts is/are present in the ready-for-use agent in an amount of from 3 to 25 % by weight.

**4.** Process according to any one of Claims 1 to 3, characterised in that the pH value of the ready-for-use agent is from 6 to 9.

**5.** Process according to any one of Claims 1 to 4, characterised in that it contains 2,3-dimercaptosuccinic acid and/or its salts as the sole keratin-reducing active substance.

**6.** Process according to any one of Claims 1 to 4, characterised in that it contains other keratin-reducing compounds in addition to the 2,3-dimercaptosuccinic acid and/or its salts.

**7.** Process according to Claim 6, characterised in that the additional keratin-reducing compound is selected from thioglycolic acid, thiolactic acid, 3-mercaptopropionic acid, cysteine or its derivatives and cysteamine or its derivatives.

**8.** Process according to Claim 7, characterised in that the shaping agent is left to take effect for from 5 to 30 minutes.

**9.** Process according to Claim 7 or 8, characterised in that the shaping agent is applied in an amount of from 60 to 120 g.

**10.** Use of an agent for the permanent shaping of hair, based on a keratin-reducing active substance which contains 2,3-dimercaptosuccinic and/or its salts as the keratin-reducing active substance.

**Revendications**

**1.** Procédé de déformation permanente des cheveux dans lequel avant ou après avoir mis les cheveux à la forme souhaitée, on les traite avec un agent de déformation, on rince à l'eau, on effectue un post-traitement oxidatif, on rince, le cas échéant on met les cheveux en plis et on les sèche, caractérisé en ce qu'on utilise, comme agent de déformation, un agent de déformation permanente des cheveux à base d'un principe actif réduisant la kératine contenant comme principe actif réduisant la kératine de l'acide 2,3-dimercaptosuccinique et/ou ses sels.

**2.** Procédé selon la revendication 1, caractérisé en ce que le sel de l'acide 2,3-dimercaptosuccinique est sélectionné parmi le sel d'ammonium et le sel de monoéthanolamine de l'acide 2,3-dimercaptosuccinique.

**3.** Procédé selon la revendication 1 ou 2, caractérisé en ce que l'acide 2,3-dimercaptosuccinique et/ou ses sels sont contenus dans l'agent prêt à l'utilisation en une quantité comprise dans la plage allant de 3 à 25 % en poids.

**4.** Procédé selon l'une des revendications 1 à 3, caractérisé en ce que la valeur pH de l'agent prête à l'utilisation est comprise dans la plage allant de 6 à 9.

**5.** Procédé selon l'une des revendications 1 à 4, caractérisé en ce que l'agent de déformation permanente contient comme seul principe actif réduisant la kératine l'acide 2,3-dimercaptosuccinique et/ou ses sels.

**6.** Procédé selon l'une des revendications 1 à 4, caractérisé en ce qu'il contient outre l'acide 2,3-dimercaptosuccinique et/ou ses sels d'autres compositions réduisant la kératine.

**7.** Procédé selon la revendication 6, caractérisé en ce que la composition supplémentaire réduisant la kératine est choisie parmi l'acide thioglycolique, l'acide thiolactique, l'acide 3-mercaptopropionique, la cystéine ou ses dérivés et cystéamine ou ses dérivés.

**8.** Procédé selon la revendication 7, caractérisé en ce que l'on laisse agir l'agent de déformation pendant une durée comprise entre 5 et 30 minutes.

9. Procédé selon la revendication 7 ou 8, caractérisé en ce que l'on applique l'agent de déformation en une quantité comprise dans la plage allant de 60 à 120 grammes.

10. Utilisation d'un agent de déformation permanente des cheveux à base d'un principe actif réduisant la kératine, contenant comme principe actif réduisant la kératine 2,3-dimercaptosuccinique et/ou ses sels.